# EUROPÄISCHE PATENTSCHRIFT

(11) **EP 4 140 318 B1**
(45) Veröffentlichungstag und Bekanntmachung des Hinweises auf die Patenterteilung: **04.11.2026**
(21) Anmeldenummer: 22192413.7
(22) Anmeldetag: 26.08.2022
(51) Int. Cl.: A23L 33/105, A23L 33/12, A23L 33/15, A23L 33/16, A61K 36/45, A61K 36/73, A61P 3/06

(54) **NORMALISIERUNG DES FETTSTOFFWECHSELS**
NORMALIZATION OF LIPID METABOLISM
NORMALISATION DU MÉTABOLISME DES LIPIDES

(30) Priorität: 26.08.2021 DE 102021122184
(43) Veröffentlichungstag der Anmeldung: 01.03.2023
(73) Patentinhaber: PM-International AG, 5445 Schengen (LU)
(72) Erfinder: SORG, Rolf, 5444 Schengen (LU); RÖHRL, Clemens, 4722 Peuerbach (AT); WEGHUBER, Julian, 4591 Molln (AT)
(74) Vertreter: Meissner Bolte Partnerschaft mbB

(56) Entgegenhaltungen:
- CN-A- 101 229 252
- KR-A- 20210 052 731
- ZHANG ZI-CHENG ET AL: "-induced hypercholesterolaemic male mice", vol. 54, no. 5, 29 November 2018 (2018-11-29), GB, pages 1576 - 1587, XP093012266, ISSN: 0950-5423, Retrieved from the Internet <URL:https://onlinelibrary.wiley.com/doi/full-xml/10.1111/ijfs.14025> DOI: 10.1111/ijfs.14025
- ARIGUNDAM USHA ET AL: "Liquid culture for efficient in vitro propagation of adventitious shoots in wild Vaccinium vitis-idaea ssp. minus (lingonberry) using temporary immersion and stationary bioreactors", SCIENTIA HORTICULTURAE, ELSEVIER, AMSTERDAM, NL, vol. 264, 16 January 2020 (2020-01-16), XP086000570, ISSN: 0304-4238, [retrieved on 20200116], DOI: 10.1016/J.SCIENTA.2020.109199
- RYYTI RIITTA ET AL: "Beneficial effects of lingonberry (Vaccinium vitis-idaea L.) supplementation on metabolic and inflammatory adverse effects induced by high-fat diet in a mouse model of obesity", PLOS ONE, vol. 15, no. 5, 7 May 2020 (2020-05-07), pages e0232605, XP093012267, DOI: 10.1371/journal.pone.0232605
- IVANOVA DIANA ET AL: "Agrimonia eupatoria tea consumption in relation to markers of inflammation, oxidative status and lipid metabolism in healthy subjects", vol. 119, no. 1, 18 October 2012 (2012-10-18), NL, pages 32 - 37, XP093012885, ISSN: 1381-3455, Retrieved from the Internet <URL:http://dx.doi.org/10.3109/13813455.2012.729844> DOI: 10.3109/13813455.2012.729844
- DUANGJAI ACHARAPORN ET AL: "Black pepper and piperine reduce cholesterol uptake and enhance translocation of cholesterol transporter proteins", JOURNAL OF NATURAL MEDICINES, vol. 67, no. 2, 27 June 2012 (2012-06-27), JP, pages 303 - 310, XP093241810, ISSN: 1340-3443, Retrieved from the Internet <URL:http://link.springer.com/article/10.1007/s11418-012-0682-7/fulltext.html> DOI: 10.1007/s11418-012-0682-7
- QIN SI ET AL: "Efficacy and safety of turmeric and curcumin in lowering blood lipid levels in patients with cardiovascular risk factors: a meta-analysis of randomized controlled trials", NUTRITION JOURNAL, vol. 16, no. 1, 11 October 2017 (2017-10-11), GB, XP093241812, ISSN: 1475-2891, Retrieved from the Internet <URL:http://link.springer.com/content/pdf/10.1186/s12937-017-0293-y.pdf> DOI: 10.1186/s12937-017-0293-y
- FUHRMAN BIANCA ET AL: "Biochemical and Molecular Action of Nutrients Ginger Extract Consumption Reduces Plasma Cholesterol, Inhibits LDL Oxidation and Attenuates Development of Atherosclerosis in Atherosclerotic, Apolipoprotein E-Deficient Mice", 1 May 2000 (2000-05-01), pages 1 - 8, XP093241817, Retrieved from the Internet <URL:https://pdf.sciencedirectassets.com/782859/1-s2.0-S0022316600X30052/1-s2.0-S0022316622140526/main.pdf?hash=369d8dc86beb4ef85e674f73f09d2120e57664da9085ae932c5f4f92d6d2754a&host=68042c943591013ac2b2430a89b270f6af2c76d8dfd086a07176afe7c76c2c61&pii=S0022316622140526&tid=spdf-60b96da3-4cc3-49d3-96c4-770>

## Beschreibung

Die Erfindung betrifft das Gebiet der Nahrungsergänzungsmittel. Insbesondere betrifft die Erfindung ein Erzeugnis zur oralen Einnahme und ein Nahrungsergänzungsmittel.

Im Blut (bzw. dem Blutplasma) vermitteln Lipoproteine den Transport wasserunlöslicher Lipide. Nach ihrer Dichte lassen sich die Lipoproteine in vier Klassen auftrennen, nämlich i) Chylomikronen, ii) VLDL (very low-density lipoproteins) oder Prä-β-Lipoproteine, iii) LDL (low-density lipoproteins) oder β-Lipoproteine und iv) HDL (high-density lipoproteins) oder α-Lipoproteine.

Die vier Lipoproteinklassen enthalten Triacylglycerole, Cholesterin und Phospholipide in charakteristischen Mengen. LDL enthält den größten Anteil an Cholesterin, nämlich etwa 50 % Cholesterin, sowie ca. 75 % Gesamtlipide. HDL enthält lediglich etwa 20 % Cholesterin sowie ca. 50 % Gesamtlipide.

Durch Endocytose und durch Bindung an LDL-Rezeptoren (LDLR), vor allem in der Leber, wird LDL-Cholesterin aus dem Blut entfernt, wodurch gleichzeitig die intrazelluläre Cholesterin-Synthese gedrosselt wird.

Ein hoher LDL-Cholesterinspiegel im Blutplasma wird als ein Risikofaktor für die Entstehung von Arteriosklerose und für einen Herzinfarkt angesehen. Ein hoher HDL-Cholesterinspiegel im Blutplasma dagegen gilt als atheroprotektiv.

Unabhängig von medizinischen Risiken kann sich ein erhöhter LDL-Cholesterinspiegel im Blutplasma oder ein geringer HDL-Cholesterinspiegel im Blutplasma in negativer Weise auf das Leben betroffener Personen auswirken. Betroffene Personen sind angehalten, ihren Lebensstil anzupassen, was sich mitunter negativ auf die subjektive Lebensqualität auswirkt. Durch eine Änderung des Lebensstiles und der Ernährung kann unter Umständen auf Medikamente verzichtet oder deren Dosis verringert werden (sogenannte supportive Verwendung "on top" zu einer Medikation).

Darüber hinaus wird die Normalisierung biologischer Parameter, insbesondere von Fettstoffwechselparametern wie HDL-Cholesterinspiegel im Blutplasma und LDL-Cholesterinspiegel im Blutplasma, unabhängig von einer medizinischen Notwendigkeit durch einen Bevölkerungsanteil angestrebt.

In den Studien von Zhang Zi-Cheng et al ("Anthocyanin extracts of lingonberry (Vaccinium vitis-idaea L.) attenuate serum lipids and cholesterol metabolism in HCDinduced hypercholesterolaemic male mice ", International Journal of Food Science and Technology, Bd. 54, Nr. 5, 29. November 2018, Seiten 1576-1587, XP093012266, GB ISSN: 0950-5423) und Arigundam Usha et al ("Liquid culture for efficient in vitro propagation of adventitious shoots in wild Vaccinium vitis-idaea ssp. minus (lingonberry) using temporary immersion and stationary bioreactors", Scientia Horticulturae, Elsevier, Amsterdam, NI, Bd. 264, 16. Januar 2020, XP086000570, ISSN: 0304-4238) wird ein cholesterinsenkender Effekt von Extrakten aus der Preiselbeere beschrieben. Auch Ryyti Riitta et al ("Beneficial effects of lingonberry (Vaccinium vitisidaea L.) supplementation on metabolic and inflammatory adverse effects induced by high-fat diet in a mouse model of obesity", PLOS ONE, Bd. 15, Nr. 5, 7. Mai 2020, Seite e0232605, XP093012267) berichten vom positiven Effekt der Preiselbeere bei der Vorbeugung von Stoffwechselerkrankungen.

In KR 2021 0052731 A wird die Verwendung von Extrakten aus der Brombeere zur Senkung der Blutfettwerte, des Cholesterins in der Leber und des Serumcholesterins beschrieben.

CN 101 229 252 A sowie Ivanova Diana et al ("Agrimonia eupatoria tea consumption in relation to markers of inflammation, oxidative status and lipid metabolism in healthy subjects", Archives of Physiology and Biochemistry, Bd. 119, Nr. 1, 18. Oktober 2012, Seiten 32-37, XP093012885, NL ISSN: 1381-3455) beschreiben den positiven Effekt von Extrakten aus dem Odermennig auf den Fettstoffwechsel und die Blutfettwerte.

Duangjai Acharaporn et al ("Black pepper and piperine reduce cholesterol uptake and enhance translocation of cholesterol transporter proteins", Journal of Natural Medicines, Bd. 67, Nr. 2, 27. Juni 2012, Seiten 303-310, XP093241810, JP ISSN: 1340-3443) untersuchten die Wirkung von schwarzem Pfeffer und des enthaltenen Piperins auf den Cholesterinstoffwechsel.

Qin Si et al ("Efficacy and safety of turmeric and curcumin in lowering blood lipid levels in patients with cardiovascular risk factors: a meta-analysis of randomized controlled trials", Nutrition Journal, Bd. 16, Nr. 1, 11. Oktober 2017, XP093241812, GB ISSN: 1475-2891) beschrieben die Wirkung von Kurkuma auf die Senkung der Blutfettwerte in Patienten mit Risikofaktoren für kardiovaskuläre Erkrankungen.

Fuhrman Bianca et al. ("Biochemical and Molecular Action of Nutrients Ginger Extract Consumption Reduces Plasma Cholesterol, Inhibits LDL Oxidation and Attenuates Development of Atherosclerosis in Atherosclerotic, Apolipoprotein E-Deficient Mice", 1. Mai 2000, Seiten 1-8, XP093241817) analysierten die Effekte eines Ingwer-Extrakts auf das Serumcholesterin, die Oxidation von LDL sowie die Prävention von Arteriosklerose.

Vor diesem Hintergrund macht sich die vorliegende Erfindung zur Aufgabe, ein Verfahren, Verwendungen und ein Erzeugnis zur oralen Einnahme anzugeben, mit denen sich der Fettstoffwechsel, insbesondere der HDL-/LDL-CholesterinStoffwechsel, normalisieren lässt, insbesondere die dazu beitragen, den LDL-Cholesterinspiegel im Blutplasma zu senken und/oder den HDL-Cholesterinspiegel im Blutplasma zu erhöhen.

Erfindungsgemäß wird die Aufgabe gelöst durch ein Erzeugnis zur oralen Einnahme, wobei das Erzeugnis einen Extrakt aus Blättern der Preiselbeerpflanze (Preiselbeerblätterextrakt), und/oder einen Extrakt aus den Blättern der Brombeerpflanze (Brombeerblätterextrakt), enthält, wobei der Extrakt oder die Extrakte durch ein Extraktionsverfahren mit einem wässrigen Extrakt hergestellt wurde. Das Erzeugnis ist ein Nahrungsergänzungsmittel und enthält pro 100 g des Erzeugnisses 4 mg bis 200 mg eines Extraktes aus dem Pfeffer (*Piper nigrum*)*.* In einer bevorzugten Ausführungsform wird das Erzeugnis oral eingenommen.

Offenbart wird (jedoch nicht als Teil der vorliegenden Erfindung) auch die nichtmedizinische Verwendung eines Extraktes aus der Preiselbeerpflanze (Preiselbeerextrakt), vorzugsweise aus Blättern der Preiselbeerpflanze (Preiselbeerblätterextrakt), und/oder eines Extraktes aus der Brombeerpflanze (Brombeerextrakt), vorzugsweise aus den aus Blättern der Brombeerpflanze (Brombeerblätterextrakt), und/oder eines Extraktes aus dem Odermennig (Odermennigextrakt), vorzugsweise aus Blättern des Odermenniges (Odermennigblätterextrakt), zur Normalisierung des Fettstoffwechsels, insbesondere des HDL-/LDL-Cholesterin-Stoffwechsels.

Offenbart wird (jedoch nicht als Teil der vorliegenden Erfindung) auch ein Erzeugnis zur oralen Einnahme zur Verwendung in einem Verfahren zur Normalisierung des Fettstoffwechsels, insbesondere des HDL-/LDL-Cholesterin-Stoffwechsels, wobei das Verfahren die orale Einnahme des Erzeugnisses zur oralen Einnahme umfasst und wobei das Erzeugnis einen Extrakt aus der Preiselbeerpflanze (Preiselbeerextrakt), vorzugsweise aus Blättern der Preiselbeerpflanze (Preiselbeerblätterextrakt), und/oder einen Extrakt aus der Brombeerpflanze (Brombeerextrakt), vorzugsweise aus Blättern der Brombeerpflanze (Brombeerblätterextrakt), und/oder einen Extrakt aus dem Odermennig (Odermennigextrakt), vorzugsweise aus Blättern des Odermenniges (Odermennigblätterextrakt), enthält.

Offenbart wird (jedoch nicht als Teil der vorliegenden Erfindung) auch ein Extrakt aus der Preiselbeerpflanze (Preiselbeerextrakt), vorzugsweise aus Blättern der Preiselbeerpflanze (Preiselbeerblätterextrakt), und/oder ein Extrakt aus der Brombeerpflanze (Brombeerextrakt), vorzugsweise aus Blättern der Brombeerpflanze (Brombeerblätterextrakt), und/oder ein Extrakt aus dem Odermennig (Odermennigextrakt), vorzugsweise aus Blättern des Odermenniges (Odermennigblätterextrakt) zur Verwendung zur Normalisierung des Fettstoffwechsels, insbesondere des HDL-/LDL-Cholesterin-Stoffwechsels.

Es wurde überraschenderweise festgestellt, dass ein Preiselbeerextrakt, insbesondere Preiselbeerblätterextrakt, ein Brombeerextrakt, insbesondere Brombeerblätterextrakt, und ein Odermennigextrakt, insbesondere Odermennigblätterextrakt eine Normalisierung des Fettstoffwechsels, insbesondere des HDL-/LDL-Cholesterin-Stoffwechsels, bewirkt.

Der Begriff "Normalisierung des Fettstoffwechsels, insbesondere des HDL-/LDL-Cholesterin-Stoffwechsels," bedeutet, dass die Konzentration mindestens eines Fettstoffwechselparameters, insbesondere LDL-Cholesterin und/oder Gesamtcholesterin und/oder HDL-Cholesterin, im Blutplasma derart verändert wird, dass eine Annäherung an einen Normbereich erfolgt, vorzugsweise ein Normbereich erreicht wird, und/oder dass die Konzentration mindestens eines Fettstoffwechselparameters, insbesondere LDL-Cholesterin und/oder Gesamtcholesterin und/oder HDL-Cholesterin, im Blutplasma innerhalb eines Normbereiches optimiert, d.h. gesenkt (Gesamtcholesterin; LDL-Cholesterin) oder erhöht (HDL-Cholesterin), wird.

Bei einem angestrebten normalen Fettstoffwechsel (mit normalem HDL-/LDL-Cholesterin-Stoffwechsel) beträgt die Konzentration an Gesamtcholesterin im Blutplasma eines Menschen < 190 mg/dL. Eine Konzentration an Gesamtcholesterin im Blutplasma eines Menschen < 190 mg/dL entspricht dem Normbereich. Für Personen mit erhöhtem Risiko für Herzkreislauferkrankungen kann auch ein erniedrigter Normbereich an Gesamtcholesterin gelten.

Bei einem angestrebten normalen Fettstoffwechsel (mit normalem HDL-/LDL-Cholesterin-Stoffwechsel) beträgt die Konzentration an LDL-Cholesterin im Blutplasma eines Menschen < 116 mg/dL. Eine Konzentration an LDL-Cholesterin im Blutplasma eines Menschen < 116 mg/dL entspricht dem Normbereich. Für Personen mit erhöhtem Risiko für Herzkreislauferkrankungen kann auch ein erniedrigter Normbereich an LDL-Cholesterin gelten.

Bei einem angestrebten normalen Fettstoffwechsel (mit normalem HDL-/LDL-Cholesterin-Stoffwechsel) beträgt die Konzentration an HDL-Cholesterin im Blutplasma eines Menschen ≥ 40 mg/dL (Männer) bzw. ≥ 48 mg/dL (Frauen). Eine Konzentration an HDL-Cholesterin im Blutplasma eines Menschen ≥ 40 mg/dL (Männer) bzw. ≥ 48 mg/dL (Frauen) entspricht dem Normbereich.

Der Begriff "Erzeugnis" stellt eine vom Menschen hergestellte Zusammensetzung dar. Der Begriff "Erzeugnis" schließt z.B. eine Frucht oder ein Blatt aus. Vorzugsweise ist das Erzeugnis ausgewählt aus einer Lösung, einer Emulsion, einer Suspension, einem Pulver, einem Granulat, einer Kapsel, einer Pastille, einer Tablette, einem Dragee, einem Sirup.

Der Ausdruck "Erzeugnis zur oralen Einnahme" bedeutet, dass das Erzeugnis zur oralen Einnahme durch den Menschen geeignet ist. Dies bedeutet auch, dass das Erzeugnis im Wesentlichen keinen Alkohol (z.B. Methanol, Ethanol, Propanol, Butanol) und kein anderes unverträgliches Lösungsmittel enthält (< 5 Gew.-%, bevorzugter < 1 Gew.-%). Mit anderen Worten, das Erzeugnis enthält insgesamt < 5 Gew.-%, bevorzugter < 1 Gew.-%, an Alkoholen und unverträglichen Lösungsmitteln.

Das Erzeugnis ist ein Nahrungsergänzungsmittel. Vorzugsweise enthält das Erzeugnis neben dem Preiselbeerblätterextrakt und/oder dem Brombeerblätterextrakt einen oder mehrere Inhaltsstoffe ausgewählt aus Vitaminen, Mineralstoffen, Aminosäuren, Fettsäuren und anderen pflanzlichen Extrakten.

Bei der Preiselbeere handelt es sich um *Vaccinium vitis-idea.* Bei der Brombeere handelt es sich um *Rubus fructicosus.* Bei dem Odermennig handelt es sich vorzugsweise um den Gemeinen Odermennig, *Agrimonia eupatoria.*

Bei dem Extrakt aus der Preiselbeerpflanze handelt es sich um einen Extrakt aus Blättern der Preiselbeerpflanze. Der Extrakt aus Blättern der Preiselbeerpflanze ist ein Extrakt, der durch ein Extraktionsverfahren mit einem wässrigen Extrakt hergestellt wurde.

Bei dem Extrakt aus der Brombeerpflanze handelt es sich um einen Extrakt aus Blättern der Brombeerpflanze. Der Extrakt aus Blättern der Brombeerpflanze ist ein Extrakt, der durch ein Extraktionsverfahren mit einem wässrigen Extrakt hergestellt wurde.

Bei dem Extrakt aus dem Odermennig handelt es sich in einer bevorzugten Ausführungsform um einen Extrakt aus Blättern des Odermenniges. Der Extrakt kann in einer Ausführungsform auch ein Extrakt aus der Frucht, optional aus der Frucht und Blättern, des Odermenniges sein.

Der Preiselbeerblätterextrakt, der Brombeerblätterextrakt und der Odermennigblätterextrakt wird durch ein Extraktionsverfahren mit einem wässrigen Extraktionsmittel aus Blättern der Preiselbeere, der Brombeere oder des Odermenniges hergestellt. Vorzugsweise enthält das wässrige Extraktionsmittel Wasser und 0,05 Gew.-% bis 10 Gew.-%, bevorzugter 0,1 Gew.-% bis 5 Gew.-%, noch bevorzugter 0,5 Gew.-% bis 3 Gew.-% einer Säure, vorzugsweise Essigsäure. Vorzugsweise beträgt im Extraktionsverfahren das Verhältnis Pflanzenmaterial (Preiselbeerblätter, Brombeerblätter oder Odermennigblätter) zu wässrigem Extraktionsmittel 1 zu 20 bis 1 zu 5. Vorzugsweise umfasst das Extraktionsverfahren das Mischen des Pflanzenmateriales mit einem siedenden wässrigen Extraktionsmittel.

Dem Fachmann sind Extraktionsverfahren zur Herstellung von Extrakten aus pflanzlichem Material unter Verwendung wässriger Extraktionsmittel im Allgemeinen bekannt.

Die Verben "enthalten" und "umfassen" und ihre Konjugation schließen auch das Verb "bestehen aus" und seine Konjugationen ein.

Bevorzugte Ausführungsformen der Erfindung können miteinander kombiniert werden, solange sich aus dem Kontext nichts Gegenteiliges ergibt. Bevorzugte Ausführungsformen sind auch in den Ansprüchen angegeben.

In einem ersten Aspekt des erfindungsgemäßen Erzeugnisses enthält das Erzeugnis den Preiselbeerblätterextrakt. Bei diesem ersten Aspekt ist in manchen Ausführungsformen bevorzugt, dass das Erzeugnis auch den Brombeerblätterextrakt und/oder den Odermennigextrakt, vorzugsweise Odermennigblätterextrakt, enthält. In einem ersten Aspekt der Verwendung wird der Preiselbeerblätterextrakt zur Normalisierung des Fettstoffwechsels, insbesondere des HDL-/LDL-Cholesterin-Stoffwechsels, verwendet. Durch die Kombination mit dem Brombeerblätterextrakt und/oder dem Odermennigextrakt, vorzugsweise Odermennigblätterextrakt, können sich die Extrakte in ihrer Wirkung unterstützen.

In einem zweiten Aspekt des erfindungsgemäßen Erzeugnisses enthält das Erzeugnis den Brombeerblätterextrakt. Bei diesem zweiten Aspekt ist in manchen Ausführungsformen bevorzugt, dass das Erzeugnis auch den Preiselbeerblätterextrakt und/oder den Odermennigextrakt, vorzugsweise Odermennigblätterextrakt, enthält. In einem zweiten Aspekt der Verwendung wird der Brombeerblätterextrakt zur Normalisierung des Fettstoffwechsels, insbesondere des HDL-/LDL-Cholesterin-Stoffwechsels, verwendet. Durch die Kombination mit dem Preiselbeerblätterextrakt und/oder dem Odermennigextrakt, vorzugsweise Odermennigblätterextrakt, können sich die Extrakte in ihrer Wirkung unterstützen.

Vorzugsweise umfasst die Normalisierung des Fettstoffwechsels, insbesondere des HDL-/LDL-Cholesterin-Stoffwechsels, eine Steigerung des HDL-Cholesterinspiegels im Blutplasma. In einer Ausführungsform ist die Normalisierung des Fettstoffwechsels, insbesondere des HDL-/LDL-Cholesterin-Stoffwechsels, die Steigerung des HDL-Cholesterinspiegels im Blutplasma.

Vorzugsweise umfasst die Normalisierung des Fettstoffwechsels, insbesondere des HDL-/LDL-Cholesterin-Stoffwechsels, eine Senkung des LDL-Cholesterinspiegels im Blutplasma. In einer Ausführungsform ist die Normalisierung des Fettstoffwechsels, insbesondere des HDL-/LDL-Cholesterin-Stoffwechsels, die Senkung des LDL-Cholesterinspiegels im Blutplasma.

Vorzugsweise umfasst die Normalisierung des Fettstoffwechsels, insbesondere des HDL-/LDL-Cholesterin-Stoffwechsels, eine Senkung des Gesamtcholesterinspiegels im Blutplasma. In einer Ausführungsform ist die Normalisierung des Fettstoffwechsels, insbesondere des HDL-/LDL-Cholesterin-Stoffwechsels, die Senkung des Gesamtcholesterinspiegels im Blutplasma.

In einer Ausführungsform umfasst die Normalisierung des Fettstoffwechsels, insbesondere des HDL-/LDL-Cholesterin-Stoffwechsels, mindestens eine Wirkung ausgewählt aus Senkung des zellulären Cholesterinspiegels, Steigerung der Expression des LDL-Rezeptors (LDLR) in Zellen, insbesondere in Leberzellen, und Steigerung der LDL-Cholesterin-Aufnahme durch Zellen, insbesondere durch Leberzellen, aus Blutplasma.

Vorzugsweise handelt es sich bei dem Verfahren und der Verwendung um ein Verfahren bzw. eine Verwendung zur Normalisierung des Fettstoffwechsels, insbesondere des HDL-/LDL-Cholesterin-Stoffwechsels, beim Menschen. In anderen Ausführungsformen handelt es sich bei dem Verfahren und der Verwendung um ein Verfahren bzw. eine Verwendung zur Normalisierung des Fettstoffwechsels, insbesondere des HDL-/LDL-Cholesterin-Stoffwechsels, bei einem Tier.

In einer Ausführungsform ist das nichtmedizinische Verfahren und die nichtmedizinische Verwendung ein nichtmedizinisches Verfahren bzw. eine nichtmedizinische Verwendung zur Normalisierung des Fettstoffwechsels, insbesondere des HDL-/LDL-Cholesterin-Stoffwechsels, bei einem Menschen, wobei der Mensch mindestens ein Medikament einnimmt, um den Fettstoffwechsel zu normalisieren. Das mindestens eine Medikament kann aus Statinen ausgewählt sein.

Vorzugsweise enthält das Erzeugnis im Wesentlichen kein organisches Lösungsmittel. Mit anderen Worten, das Erzeugnis enthält vorzugsweise < 5 Gew.-%, bevorzugter < 1 Gew.-%, organisches Lösungsmittel.

In einer Ausführungsform enthält das Erzeugnis pro 100 g des Erzeugnisses 25 mg bis 250 mg Lutein.

In einer Ausführungsform enthält das Erzeugnis pro 100 g des Erzeugnisses 2 mg bis 12 mg Zeaxanthin.

In einer Ausführungsform enthält das Erzeugnis pro 100 g des Erzeugnisses 1000 mg bis 5000 mg Docosahexaensäure.

In einer Ausführungsform enthält das Erzeugnis pro 100 g des Erzeugnisses 25 mg bis 200 mg, bevorzugter 40 mg bis 130 mg, besonders bevorzugt 60 mg bis 90 mg, Eicosapentaensäure.

In einer Ausführungsform enthält das Erzeugnis pro 100 g des Erzeugnisses 100 mg bis 1200 mg Coenzym Q10.

In einer Ausführungsform enthält das Erzeugnis pro 100 g des Erzeugnisses 0,1 g bis 10 g, bevorzugter 0,3 g bis 7 g, besonders bevorzugt 0,4 g bis 6 g, Extrakt aus rotem Ginseng (Panax ginseng).

In einer Ausführungsform enthält das Erzeugnis pro 100 g des Erzeugnisses 150 mg bis 3000 mg, bevorzugter 500 mg bis 1500 mg, besonders bevorzugt 750 mg bis 1250 mg, an Isoflavonen, vorzugsweise an Soyaisoflavonen.

In einer Ausführungsform enthält das Erzeugnis pro 100 g des Erzeugnisses 30 g bis 90 g, bevorzugter 40 g bis 80 g, besonders bevorzugt 50 g bis 70 g, Gummi arabicum.

In einer Ausführungsform enthält das Erzeugnis neben dem Extrakt aus Blättern der Preiselbeerpflanze und/oder dem Extrakt aus Blättern der Brombeerpflanze, wobei der Extrakt oder die Extrakte durch ein Extraktionsverfahren mit einem wässrigen Extrakt hergestellt wurde, und dem Extrakt aus dem Pfeffer einen oder mehrere Inhaltsstoffe ausgewählt aus Zink, Biotin, Omega-3-Fettsäuren, Coenzym Q10, Berberin, Lutein, Zeaxanthin, einem Extrakt aus Broccoli, einem Extrakt aus der Zwiebel, einem Extrakt aus dem Apfel, einem Extrakt aus der Acerola, einem Extrakt aus der Tomate, einem Extrakt aus der Kurkuma, einem Extrakt aus dem Knoblauch, einem Extrakt aus dem Basilikum, einem Extrakt aus dem Oregano, einem Extrakt aus dem Zimtbaum, einem Extrakt aus der Karotte, einem Extrakt aus dem Holunder, einem Extrakt aus der Johannisbeere, einem Extrakt aus der Heidelbeere, einem Extrakt aus der Himbeere, einem Extrakt aus der Apfelbeere, einem Extrakt aus dem Spinat, einem Extrakt aus der Kirsche, einem Extrakt aus der Heidelbeere und einem Extrakt aus dem Rosenkohl.

In einer Ausführungsform enthält das Erzeugnis neben dem Extrakt aus Blättern der Preiselbeerpflanze und/oder dem Extrakt aus Blättern der Brombeerpflanze und dem Extrakt aus dem Pfeffer Zink und/oder Biotin.

In einer Ausführungsform enthält das Erzeugnis neben dem Extrakt aus Blättern der Preiselbeerpflanze und/oder dem Extrakt aus Blättern der Brombeerpflanze und dem Extrakt aus dem Pfeffer Omega-3-Fettsäuren, bevorzugt Docosahexaensäure und/oder Eicosapentaensäure.

In einer Ausführungsform enthält das Erzeugnis pro 100 g des Erzeugnisses ein oder mehrere Bestandteile, die ausgewählt sind aus 0,25 mg bis 2 g einer Curcuminverbindung, 4 mg bis 200 mg eines Extraktes aus dem Ingwer (*Zingiber officinale)* und 0,4 g bis 6 g eines Extraktes aus rotem Ginseng (Panax ginseng). In einer Ausführungsform enthält das Erzeugnis pro 100 g des Erzeugnisses 0,25 mg bis 2 g einer Curcuminverbindung, 4 mg bis 200 mg eines Extraktes aus dem Ingwer (*Zingiber officinale*) und 0,4 g bis 6 g eines Extraktes aus rotem Ginseng (Panax ginseng). Vorzugsweise ist der Extrakt aus dem Pfeffer (*Piper nigrum*) Piperin. Bei der Curcuminverbindung handelt es sich bevorzugt um einen Curcumin-Cyclodextrin-Komplex, insbesondere einen Curcumin-Gamma-Cyclodextrin-Komplex. Bevorzugt handelt es sich bei dem Extrakt aus dem Ingwer um einen Extrakt aus dem Rhizom des Ingwers (*Zingiber officinale).* Diese Substanzen wirken als Bioenhancer und steigern die Bioverfügbarkeit der Inhaltsstoffe des Erzeugnisses.

In einer Ausführungsform enthält das Erzeugnis pro 100 g des Erzeugnisses mindestens 5 g, bevorzugter mindestens 10 g, noch bevorzugter mindestens 15 g, Kohlenhydrate.

In einer Ausführungsform enthält das Erzeugnis ein oder mehrere Bestandteile, die ausgewählt sind aus einem Extrakt aus grünem Tee (vorzugsweise *Camellia sinensis),* einem Extrakt aus dem Kaffee (vorzugsweise *Coffea arabica),* einem Extrakt aus der Sauerkirsche (vorzugsweise *Prunus cerasus),* einem Extrakt aus dem Grünkohl (vorzugsweise *Brassica oleracea acephala),* einem Extrakt aus der Kurkuma (vorzugsweise *Curcuma longa),* einem Extrakt aus dem Broccoli (vorzugsweise *Brassica oleracea italica)* und einem Extrakt aus der Heidelbeere (vorzugsweise *Vaccinium corymbosum).*

In einer Ausführungsform enthält das Erzeugnis einen Extrakt aus grünem Tee (vorzugsweise *Camellia sinensis),* einen Extrakt aus dem Kaffee (vorzugsweise *Coffea arabica),* einen Extrakt aus der Sauerkirsche (vorzugsweise *Prunus cerasus),* einen Extrakt aus dem Grünkohl (vorzugsweise *Brassica oleracea acephala),* einen Extrakt aus der Kurkuma (vorzugsweise *Curcuma longa),* einen Extrakt aus dem Broccoli (vorzugsweise *Brassica oleracea italica)* und einen Extrakt aus der Heidelbeere (vorzugsweise *Vaccinium corymbosum).*

Der Extrakt aus dem Kaffee (vorzugsweise *Coffea arabica)* ist vorzugsweise ein Extrakt aus der Bohne des Kaffees, besonders bevorzugt aus der grünen Bohne. In dieser Ausführungsform enthält das Erzeugnis vorzugsweise pro 100 g 2,3 mg bis 5 g, bevorzugter 4,14 mg bis 3 g des Extraktes aus dem Kaffee (vorzugsweise *Coffea arabica).* Der Extrakt aus der Sauerkirsche (vorzugsweise *Prunus cerasus)* ist vorzugsweise ein Extrakt aus der Kirsche der Sauerkirsche. In dieser Ausführungsform enthält das Erzeugnis vorzugsweise pro 100 g 0,025 mg bis 2 g, bevorzugter 0,045 mg bis 1,5 g des Extraktes aus der Sauerkirsche. Der Extrakt aus dem Grünkohl (vorzugsweise *Brassica oleracea acephala)* ist vorzugsweise ein Extrakt aus Blättern des Grünkohls. In dieser Ausführungsform enthält das Erzeugnis vorzugsweise pro 100 g 0,025 mg bis 2 g, bevorzugter 0,045 mg bis 1,5 g des Extraktes aus dem Grünkohl. Der Extrakt aus der Kurkuma (vorzugsweise *Curcuma longa)* ist vorzugsweise ein Extrakt aus der Wurzel der Kurkuma. In dieser Ausführungsform enthält das Erzeugnis vorzugsweise pro 100 g 0,25 mg bis 2 g, bevorzugter 0,45 mg bis 1,5 g, des Extraktes aus der Kurkuma. Der Extrakt aus dem Broccoli (vorzugsweise *Brassica oleracea italica)* ist vorzugsweise ein Extrakt aus dem Röschen des Broccolis. In dieser Ausführungsform enthält das Erzeugnis vorzugsweise pro 100 g 0,025 mg bis 2 g, bevorzugter 0,045 mg bis 1,5 g, des Extraktes aus dem Broccoli. Der Extrakt aus der Heidelbeere (vorzugsweise *Vaccinium corymbosum)* ist vorzugsweise ein Extrakt der Frucht der Heidelbeere. In dieser Ausführungsform enthält das Erzeugnis vorzugsweise pro 100 g 0,025 mg bis 2 g, bevorzugter 0,045 mg bis 1,5 g, des Extraktes aus der Heidelbeere. Der Extrakt aus grünem Tee (vorzugsweise *Camellia sinensis)* ist vorzugsweise ein Extrakt aus Blättern des grünen Tees. In dieser Ausführungsform enthält das Erzeugnis vorzugsweise pro 100 g 4,14 mg bis 3000 mg des Extraktes aus grünem Tee.

In einer Ausführungsform enthält das Erzeugnis ein oder mehrere Bestandteile, die ausgewählt sind aus einem Extrakt aus Broccoli (vorzugsweise *Brassica oleracea italica),* insbesondere aus Samen, Keimen, Röschen, Stielen und/oder Sprossen des Broccolis, einem Extrakt aus der Zwiebel (vorzugsweise *Allium cepa),* insbesondere aus der Zwiebel der Zwiebel, einem Extrakt aus dem Apfel (vorzugsweise *Malus domestica),* insbesondere aus der Apfelfrucht, insbesondere aus Schalen der Apfelfrucht, einem Extrakt aus der Acerola (vorzugsweise *Malpighia glabra),* insbesondere aus der Frucht der Acerola, einem Extrakt aus der Tomate (vorzugsweise *Lycopersicon esculentum),* insbesondere aus der Frucht der Tomate, einem Extrakt aus der Kurkuma (vorzugsweise *Curcuma longa),* insbesondere aus der Wurzel der Kurkuma, einem Extrakt aus dem Knoblauch (vorzugsweise *Allium sativum),* insbesondere aus der Knoblauchzehe, einem Extrakt aus dem Basilikum (vorzugsweise *Ocimum basilicum),* insbesondere aus Blättern des Basilikums, einem Extrakt aus dem Oregano (vorzugsweise *Origanum vulgare),* insbesondere aus Blättern des Oreganos, einem Extrakt aus dem Zimtbaum (vorzugsweise *Cinnamonum cassia),* insbesondere aus der Rinde des Zimtbaumes, einem Extrakt aus der Karotte (vorzugsweise *Dacus carota),* insbesondere aus der Wurzel der Karotte, einem Extrakt aus dem Holunder (vorzugsweise *Sambucus nigra),* insbesondere aus der Frucht des Holunders, einem Extrakt aus der Johannisbeere (vorzugsweise *Ribes),* insbesondere der schwarzen Johannisbeere (vorzugsweise *Ribes nigrum),* insbesondere aus der Frucht der schwarzen Johannisbeere, einem Extrakt aus der Heidelbeere (vorzugsweise *Vaccinium),* insbesondere aus der Frucht der Heidelbeere, einem Extrakt aus der Himbeere (vorzugsweise *Rubus idaeus),* insbesondere aus der Frucht der Himbeere, einem Extrakt aus der Apfelbeere (vorzugsweise *Aronia),* insbesondere aus der Frucht der Apfelbeere, einem Extrakt aus dem Spinat (vorzugsweise *Spinacia oleracea),* insbesondere aus den Blättern des Spinates, einem Extrakt aus der Kirsche (vorzugsweise *Prunus avium),* insbesondere aus der Kirschfrucht, einem Extrakt aus der Heidelbeere (vorzugsweise *Vaccinium),* insbesondere aus der Frucht der Heidelbeere, und einem Extrakt aus dem Rosenkohl (vorzugsweise *Brassica oleracea gemmifera),* insbesondere aus Röschen des Rosenkohles.

Vorzugsweise enthält das Erzeugnis pro 100 g des Erzeugnisses von diesen Extrakten insgesamt 0,10 g bis 0,75 g. Bei diesen Extrakten kann es sich um einen eingetrockneten Auszug aus entsprechenden Pflanzenteilen handeln, wobei der Auszug mit Hilfe eines organischen Lösungsmittels und/oder Wasser und mit einem Droge-Extrakt-Verhältnis zwischen 1:120 und 1:10 gewonnen wird. Bei diesen Extrakten kann es sich auch um ein Konzentrat handeln, ein entwässertes Produkt aus entsprechenden Pflanzenteilen. Die Extrakte weisen einen hohen Gehalt an wertvollen, pflanzlichen Sekundärmetaboliten wie Polyphenolen auf.

In einer Ausführungsform enthält das Erzeugnis pro 100 g des Erzeugnisses ein oder mehrere Bestandteile, die ausgewählt sind aus 100 mg bis 1500 mg Niacin, 20 mg bis 270 mg Pantothensäure, 5 mg bis 100 mg Pyridoxin, 4 mg bis 72 mg Riboflavin, 5 mg bis 70 mg Thiamin, 10 µg bis 10 mg Cyanocobolamin, 0,5 mg bis 7 mg Biotin und 0,5 mg bis 12 mg Folsäure. In einer Ausführungsform enthält das Erzeugnis pro 100 g des Erzeugnisses 100 mg bis 1500 mg Niacin, 20 mg bis 270 mg Pantothensäure, 5 mg bis 100 mg Pyridoxin, 4 mg bis 72 mg Riboflavin, 5 mg bis 70 mg Thiamin, 10 µg bis 10 mg Cyanocobolamin, 0,5 mg bis 7 mg Biotin und 0,5 mg bis 12 mg Folsäure.

In einer Ausführungsform enthält das Erzeugnis pro 100 g des Erzeugnisses 1500 µg bis 4000 µg Retinol-Äquivalente und/oder 60 mg bis 120 mg a-Tocopherol-Äquivalente.

In einer Ausführungsform enthält das Erzeugnis pro 100 g des Erzeugnisses 0,3 g bis 3 g Vitamin C.

Nachfolgend wird die Erfindung an Hand von Beispielen weiter erläutert.

### a) Protokoll zur Herstellung des Preiselbeerblätterextraktes, Brombeerblätterextraktes und Odermennigblätterextraktes:

Getrocknete Blätter der Preiselbeere *(Vaccinium vitis-idea),* getrocknete Blätter der Brombeere *(Rubus fructicosus)* und getrocknete Blätter des gemeinen Odermennigs *(Agrimonia eupatoria)* wurden aus einer Apotheke bezogen. 90 ml destillierten Wassers wurden auf einer Wärmeplatte aufgekocht. Essigsäure (Endkonzentration 1 %) wurde zugegeben, um die Quellung des Pflanzenmaterials zu verhindern. 10 g Pflanzenmaterial (getrocknete Blätter der Preiselbeere *(Vaccinium vitis-idea),* der Brombeere *(Rubus fructicosus)* oder des gemeinen Odermennigs *(Agrimonia eupatoria))* wurden zugegeben und die Suspension wurde 20 Minuten lang ohne weitere Erwärmung auf der Wärmeplatte gerührt. Anschließend wurde das Endvolumen mit destilliertem Wasser auf 100 ml eingestellt und die Suspension wurde 15 Minuten lang im Ultraschallwasserbad beschallt. Nach der Zentrifugation (1.700 g, 10 min, Raumtemperatur) wurde der Überstand durch einen Faltenfilter filtriert, gefolgt von einem weiteren Klärungsschritt durch Zentrifugation (10.000 g, 2 min, Raumtemperatur). Die Überstände wurden steril gefiltert (PES-Membran, 0,45 µm) und in Aliquots bei -20 °C gelagert. Die Trockensubstanz wurde mit dem MA160 Moisture Analyzer (Sartorius, Göttingen, Deutschland) bestimmt und betrug 12 mg/mL (Odermennigblätterextrakt), 26,3 mg/ml (Preiselbeerblätterextrakt) und 17,2 mg/ml (Brombeerblätterextrakt).

### b) Protokoll zum Kultivieren von Zellen für nachfolgende Experimente

Die Zelllinien wurden unter Standardkultivierungsbedingungen gehalten und routinemäßig auf Mykoplasmeninfektionen überprüft. Die Zellen wurden in allen Experimenten innerhalb von 15 Passagen verwendet. HepG2-Zellen (ATCC: HB-8065) wurden in MEM (Minimal Essential Medium) kultiviert, das mit 10 % fetalem Kälberserum (FBS), 1 % Penicillin/Streptomycin und 1 % nicht-essentiellen Aminosäuren (alle von Biochrom GmbH Berlin, Deutschland) ergänzt wurde. Huh-7-Zellen (ATCC: JCRB-0403) wurden in DMEM (Dulbecco's Modified Eagle's Medium) mit 10 % FBS und 1 % Penicillin/Streptomycin (Biochrom) kultiviert.

Vor den experimentellen Behandlungen wurden die Zellen einmal mit calcium- und magnesiumhaltiger, phosphatgepufferter Salzlösung (PBS^{+/+}; Biochrom) gewaschen. Pflanzenblätterextrakte und Lovastatin (Sigma-Aldrich, Saint Louis, Missouri, USA) wurden unter serumreduzierten Bedingungen (Medien mit 1 % FBS) verabreicht.

### c) Protokoll für qRT-PCR und Immunoblotting

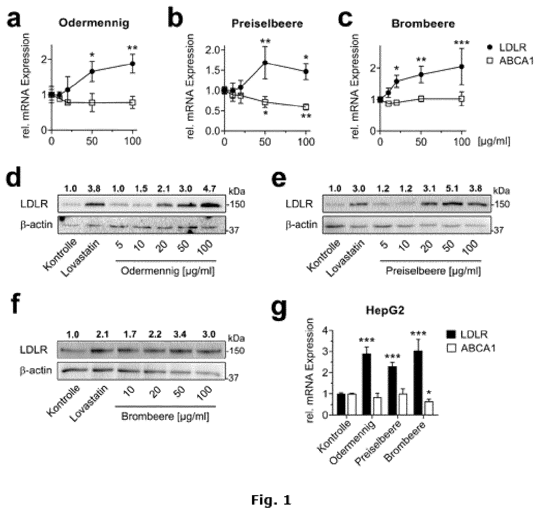

Die Zellen (Huh-7: 2 x 10⁵ und HepG2 5 x 10⁵ Zellen/Well) wurden am Tag 0 in 6-Well-Platten ausgesät. Am Tag 2 wurden die Zellen mit PBS^{+/+} gewaschen und mit Preiselbeerblätterextrakt, Brombeerblätterextrakt, Odermennigblätterextrakt oder Lovastatin unter serumreduzierten Bedingungen (1 % FBS) behandelt. Die Blätterextrakte wurden in den angegebenen Konzentrationen eingesetzt (siehe Fig. 1). Lovastatin (10 µM, verdünnt in DMSO) wurde als Positivkontrolle verwendet. Die endgültige DMSO-Konzentration betrug 0,1 % und hatte keinen Einfluss auf die LDL-Rezeptor-Expression (Daten nicht gezeigt). Nach 24 Stunden wurden die Zellen für die RNA-Isolierung oder Proteinisolierung aufbereitet.

RNA-Isolierung, cDNA-Synthese und qRT-PCR wurden nach Standardmethoden unter Verwendung von Taqman-Sonden (Thermo Fisher Scientific) gegen den LDL-Rezeptor (Hs00181192_m1) und ABCA1 (Hs00194045_m1) durchgeführt. Die Expression wurde auf zwei Hauskeeping-Gene, ACTB (Hs99999903_m1) und GAPDH (Hs99999905_m1), normalisiert.

Die Proteine wurden mit Cell Lysis Buffer (Cell Signaling Technology, Danvers, MA, USA) nach den Anweisungen des Herstellers isoliert. Die Proteinkonzentrationen wurden mit dem Micro BCA Protein Assay Kit (Thermo Fisher Scientific) bestimmt. Zwanzig µg Protein wurden mit Laemmli-Probenpuffer, der Mercaptoethanol enthielt, ohne Kochen gemischt und durch SDS-PAGE (Trenngel: 7,5 % Acrylamid) aufgetrennt. Anschließend wurden die Proben auf eine PVDF-Membran (0,2 µm; Bio-Rad Laboratories; Hercules, CA, US) geblottet. Als Reagenz zum Blockieren, Waschen und Verdünnen der Antikörper wurde Western Blocking Reagent (Roche Diagnostics, Mannheim, Deutschland) gemäß den Anweisungen des Herstellers verwendet. Die Membranen wurden mit primären Antikörpern gegen LDLR [1/400; Christian Wadsack, et al., Intrauterine growth restriction is associated with alterations in placental lipoprotein receptors and maternal lipoprotein composition, American journal of physiology. Endocrinology and metabolism, 2007, 292, E476-84] und β-Actin (1/1000; Cell Signaling Technology #4967S, Charge #11) über Nacht bei 4 °C inkubiert. Die Membranen wurden mit den entsprechenden Peroxidase-markierten sekundären Antikörpern inkubiert, und die Färbung wurde durch Chemilumineszenz mit dem Clarity Max Western ECL Substrat und einem ChemiDoc MP Imager (beide von Bio-Rad Laboratories) nachgewiesen. Die Bandenintensitäten wurden semiquantitativ durch Densitometrie mit Image J (NIH, USA; Version 1.52d) analysiert.

### d) Untersuchung der Wirkung von Preiselbeerblätterextrakt, Brombeerblätterextrakt und Odermennigblätterextrakt auf die Expression von LDL-Rezeptor-mRNA und des LDL-Rezeptorproteins

Durch Bindung an LDL-Rezeptoren, vor allem in der Leber, wird LDL-Cholesterin aus dem Blut entfernt.

Daher wurde untersucht, ob der Preiselbeerblätterextrakt, der Brombeerblätterextrakt und der Odermennigblätterextrakt die Expression von LDL-Rezeptor-mRNA und des LDL-Rezeptorproteins in Leberzellen steigern.

Fig. 1 zeigt, dass der Preiselbeerblätterextrakt, der Brombeerblätterextrakt und der Odermennigblätterextrakt die Expression von LDL-Rezeptor-mRNA und des LDL-Rezeptorproteins in Huh-7-Zellen (humane Leberzellen) sogar in stärkerem Maße bewirken können als der Wirkstoff Lovastatin (einem Statin, das zur Behandlung der Hypercholesterinämie eingesetzt wird).

Fig. 1 a-f) zeigen Ergebnisse von Versuchen, bei denen Huh-7-Zellen 24 Stunden lang mit den angegebenen Konzentrationen der wässrigen Pflanzenextrakte (Preiselbeerblätterextrakt, Brombeerblätterextrakt und Odermennigblätterextrakt) unter serumreduzierten Bedingungen (1 % FBS) behandelt wurden.

Die LDL-Rezeptor- und ABCA1-mRNA-Expression wurde mittels qRT-PCR quantifiziert. Die Ergebnisse sind in Fig. 1 a-c) dargestellt (es handelt sich um gepoolte Daten von zwei unabhängigen Experimenten, die in doppelter Ausführung durchgeführt wurden).

Die LDL-Rezeptor-Proteinexpression wurde mittels Immunoblot analysiert. Die Ergebnisse sind in Fig. 1 d-f) dargestellt. Lovastatin (10 µM) diente als Positivkontrolle. Der Faktor der relativen Veränderung der Proteinexpression wurde jeweils aus zwei bis drei unabhängigen Experimenten berechnet und ist jeweils über den Banden angegeben.

Der Preiselbeerblätterextrakt in einer Konzentration von 50 µg/ml induzierte die LDLR-mRNA- und Proteinexpression um das 1,7- bzw. 5,1-fache (Abbildung 1b und e). Die Expression von ABCA1, das durch erhöhte zelluläre Cholesterinspiegel positiv reguliert wird und der Wirkung des LDL-Rezeptorproteins entgegenwirkt, indem es Cholesterin zu extrazellulären Akzeptoren exportiert, wurde verringert (Abbildung 1b und e). Der Brombeerblätterextrakt induzierte die LDL-Rezeptor-mRNA ebenfalls dosisabhängig, während die ABCA1-Expression unverändert blieb (Abbildung 1c). Die LDL-Rezeptor-Proteinexpression war bei 50 µg/ml um das 3,4-Fache erhöht (Abbildung 1f). Der Odermennigblätterextrakt induzierte dosisabhängig die Expression von LDL-Rezeptor-mRNA sowie von Protein (Fig. 1a und d). Die Expression von ABCA1, das durch erhöhte zelluläre Cholesterinspiegel positiv reguliert wird und der Wirkung von LDL-Rezeptor entgegenwirkt, indem es Cholesterin zu extrazellulären Akzeptoren exportiert, wurde tendenziell verringert.

Um auszuschließen, dass die Wirkungen auf eine einzelne Zelllinie beschränkt sind, wurden die Extrakte auf HepG2-Zellen angewendet, eine andere aus menschlichen Hepatozyten gewonnene Zelllinie, die verschiedene Aspekte des Cholesterin- und Lipoproteinstoffwechsels beibehält.

Fig. 1 g) zeigt Ergebnisse eines Versuches, bei dem HepG2-Zellen (Leberzellen) mit Pflanzenblätterextrakten (Odermennig: 50 µg/ml, Preiselbeere: 25 µg/ml, Brombeere: 100 µg/ml Trockenmasse) unter serumreduzierten Bedingungen (1% FBS) 24 Stunden lang behandelt wurden. Die Genexpression wurde mittels qRT-PCR quantifiziert. Dargestellt sind die gepoolten Daten von zwei unabhängigen Experimenten, die in doppelter Ausführung durchgeführt wurden.

Im Vergleich zu Huh-7-Zellen induzierten alle getesteten Extrakte die Expression der LDL-Rezeptor-mRNA um das ca. 3-fache, während die ABCA1-Expression unverändert oder leicht verringert war (Abbildung 1g).

Insgesamt deuten diese Daten darauf hin, dass Extrakte aus Preiselbeer-, Brombeer-und Odermennigblättern die LDL-Rezeptor-Expression in Leberzellen erhöhen.

### e) Untersuchung der Veränderung der zellulären Cholesterinhomöostase

In einem weiteren Schritt wurden die Blätterextrakte hinsichtlich ihrer Wirkung auf die Veränderung des zellulären Cholesterinhaushaltes untersucht. Die Verarmung an zellulärem Cholesterin ist die Grundlage für die kompensatorische Induktion der LDLR-Expression. Daher wurde der zelluläre Cholesterinspiegel als Reaktion auf die Behandlung mit dem Brombeerblätterextrakt, Preiselbeerblätterextrakt und Odermennigblätterextrakt quantifiziert.

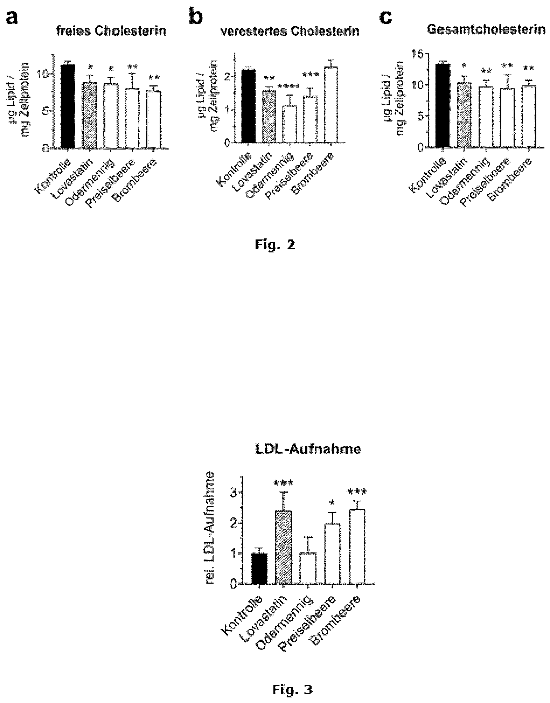

Freies Cholesterin, das nicht zu Fettsäuren verestert ist und überwiegend in Zellmembranen lokalisiert ist, machte unter den Versuchsbedingungen den größten Teil des zellulären Cholesterins in Huh-7-Zellen aus. Die Ergebnisse sind in Fig. 2a-c) dargestellt. Dargestellt sind die gepoolten Daten von zwei unabhängigen Experimenten, die in doppelter Ausführung durchgeführt wurden.

Die Menge an zellulärem freien Cholesterin wurde durch die Extrakte signifikant reduziert, und zwar in einem Ausmaß, das mit dem von Lovastatin vergleichbar oder größer ist (siehe Fig. 2a). Die Menge an Cholesterinestern, die aus mit Fettsäuren verestertem Cholesterin bestehen und die Speicherform von Cholesterin in Lipidtröpfchen darstellen, wurden durch den Odermennigblätterextrakt und den Preiselbeerblätterextrakt, nicht aber durch den Brombeerblätterextrakt verringert (siehe Fig. 2b). Der Gesamtcholesteringehalt (d. h. freies Cholesterin plus verestertes Cholesterin) sank nach der Inkubation mit Pflanzenextrakten um ~ 30 % (siehe Fig. 2c). Diese Daten deuten darauf hin, dass die nach der Behandlung mit diesen Extrakten beobachtete Zunahme der LDLR-Expression tatsächlich auf eine Verringerung des zellulären Cholesterinspiegels zurückzuführen ist.

### Versuchsprotokoll:

Der Gehalt an freiem und verestertem Cholesterin wurde direkt durch Gaschromatographie analysiert (S. Vondra, et al., Metabolism of cholesterol and progesterone is differentially regulated in primary trophoblastic subtypes and might be disturbed in recurrent miscarriages, Journal of lipid research, 2019, 60, 1922-1934).

Huh-7-Zellen wurden am Tag 0 in 6-cm-Schalen mit einer Dichte von 5 x 10⁵ Zellen/Schale ausgesät. Am zweiten Tag wurden die Zellen mit PBS^{+/+} gewaschen und 24 Stunden lang mit Lovastatin (10 µM) oder Pflanzenextrakten (Preiselbeerblätterextrakt: 50 µg/ml, Brombeerblätterextrakt: 100 µg/ml, Odermennigblätterextrakt: 100 µg/ml Trockenmasse) unter serumreduzierten Bedingungen (1 % FBS) inkubiert. Anschließend wurden die Zellen gewaschen, mit Trypsin/EDTA abgelöst, in PBS resuspendiert und zentrifugiert (4 °C, 200 g, 5 min).

Die Lipide wurden durch die Standard-Folch-Extraktion aus dem Zellpellet isoliert. Ein Aliquot des Pellets wurde für die Bestimmung des Zellproteins mit dem Bradford-Assay verwendet. Die Lipide wurden mit einem Gaschromatographen GC-2010 (Shimadzu, Kyoto, Japan) aufgetrennt, der mit einem Injektor mit programmierter Temperatur (PTV), einer Kapillarsäule ZB-5HT (15 m × 0,32 mm × 0,1 µm; Phenomenex, Aschaffenburg, Deutschland) und einem FID-Detektor ausgestattet war. Tridecanoylglycerin und Cholesterylmyristat (beide von Sigma-Aldrich) wurden als Standards für freies bzw. verestertes Cholesterin verwendet. Die Chromatogramme wurden mit GC Solutions 2.3 (Shimadzu) analysiert, und die Werte wurden auf das Zellprotein normiert.

### f) Untersuchung der LDL-Aufnahme durch Zellen

Statine bewirken eine erhöhte LDLR-vermittelte Clearance von zirkulierendem LDL-Cholesterin in der Leber, was ihre wichtigste atheroprotektive Wirkung darstellt. Die Aufnahme von menschlichem LDL-Cholesterin wurde daher in Huh-7-Zellen nach Inkubation mit den Pflanzenblätterextrakten gemessen.

Das Ergebnis ist in Fig. 3 illustriert. Die LDL-Cholesterin-Aufnahme war nach der Behandlung mit Lovastatin um das 2,3-Fache erhöht. Extrakte aus Preiselbeer- und Brombeerblättern führten zu einem vergleichbaren Anstieg der LDL-Cholesterin-Aufnahme.

Zusammengenommen senken Pflanzenextrakte aus Preiselbeer- und Brombeerblättern den zellulären Cholesterinspiegel, erhöhen die LDLR-mRNA- und Proteinexpression und steigern die LDL-Aufnahme in vergleichbarer Weise wie Lovastatin.

### Versuchsbeschreibung der Messung der zellulären LDL-Aufnahme:

Zur Messung der zellulären Aufnahme von fluoreszenzmarkiertem LDL wurde ein von A. Loregger et al. veröffentlichtes Protokoll mit Änderungen angewendet (A. Loregger et al., Assaying Low-Density-Lipoprotein (LDL) Uptake into Cells, Methods in molecular biology, 2017, 1583, 53-63). Zunächst wurde LDL von gesunden normolipidämischen Probanden durch sequenzielle Flotationsultrazentrifugation isoliert (Verne N. Schumaker und Donald L. Puppione, in Plasma Lipoproteins Part A: Preparation, Structure, and Molecular Biology, Elsevier, 1986, pp. 155-170). Ein mg LDL (verdünnt auf 2 mg/ml in PBS) wurde mit 50 µg DyLight 488 NHS-Ester (Thermo Fisher Scientific) in Gegenwart von NaHCO₃ (Endkonzentration: 100 mM) bei Raumtemperatur für 1 Stunde markiert. Ungebundener Farbstoff wurde durch ausgiebige Dialyse gegen PBS entfernt. Huh-7-Zellen wurden am Tag 0 in 24-WellPlatten mit einer Dichte von 5 x 10⁴ Zellen/Well ausgesät. Am zweiten Tag wurden die Zellen mit PBS^{+/+} gewaschen und 24 Stunden lang mit Lovastatin (10 µM) oder Pflanzenextrakten (Preiselbeerblätterextrakt: 50 µg/ml, Brombeerblätterextrakt: 100 µg/ml, Odermennigblätterextrakt 100 µg/ml, Trockenmasse) unter serumreduzierten Bedingungen (1 % FBS) inkubiert. An Tag 3 wurden die Zellen zweimal mit PBS^{+/+} gewaschen und 2 Stunden lang mit 10 µg/ml DyLight-LDL in einem Medium mit 0,5% BSA inkubiert. Wells, die neben 10 µg/ml DyLight-LDL auch 200 µg/ml unmarkiertes LDL enthielten, dienten als Kontrollen zur Bestimmung der spezifischen LDL-Aufnahme. Nach 2 Stunden wurden die Zellen gewaschen, lysiert und wie von A. Loregger et al. beschrieben verarbeitet. Die spezifische LDL-Aufnahme wurde durch Subtraktion der Fluoreszenz von Wells mit überschüssigem unmarkiertem LDL berechnet. Die Werte wurden auf das mit dem Micro BCA Protein Assay Kit bestimmte Zellprotein normiert.

### g) In vivo-Versuche

Männliche C57BL/6-Mäuse (acht Wochen alt, sechs Mäuse pro Gruppe) wurden vom Zentrum für Biomedizinische Forschung (Medizinische Universität Wien) bezogen und unter Standard-Laborbedingungen untergebracht. Die Mäuse erhielten Wasser und Nagerfutter ad libitum. Der Preiselbeerblätterextrakt oder der Brombeerblätterextrakt oder Wasser wurden fünf Tage lang täglich oral verabreicht (250 µl/Maus). Das Gesamtcholesterin wurde mit dem CHOD-PAP-Kit (Greiner Bio-One) aus dem Blutplasma bestimmt. HDL-Cholesterin wurde mit dem HDL-C Immuno FS Kit (DiaSys, Holzheim, Deutschland) aus dem Blutplasma bestimmt. Das als VLDL oder LDL vorliegende Cholesterin wurde durch Subtraktion des HDL-Cholesterins vom Gesamtcholesterin berechnet. Die Expression von LDLR und ABCA1 wurde in Leberproben bestimmt.

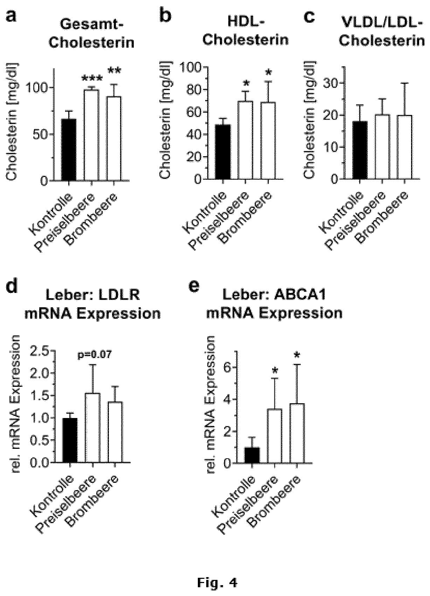

Die Ergebnisse sind in Fig. 4 dargestellt. Unerwarteterweise war das Gesamtplasmacholesterin nach der Behandlung mit beiden Extrakten erhöht (Fig. 4a). Dieser Anstieg war auf eine Zunahme des Cholesterins zurückzuführen, das über das HDL transportiert wird (Fig. 4b), das der Haupttransporteur von Cholesterin in Mäusen ist. Mit anderen Worten, HDL-Cholesterin war nach der Behandlung mit beiden Extrakten erhöht (Fig. 4b). Das restliche Cholesterin - d. h. das über VLDL und LDL transportierte Cholesterin - wurde nicht verändert (Fig. 4c). Verglichen mit der LDLRsteigernden Wirkung in vitro, erhöhten beide Extrakte die LDLR-mRNA-Expression in der Mäuseleber nur leicht (Fig. 4d, qRT-PCR). Auch das LDLR-Protein wurde nicht signifikant verändert. Die Expression von ABCA1 in der Leber war jedoch deutlich erhöht (Fig. 4e, qRT-PCR), was mit den erhöhten HDL-Cholesterinspiegeln bei Mäusen, die mit Extrakten aus Preiselbeer- oder Brombeerblättern behandelt wurden, übereinstimmt.

### h) Allgemeines zur Statistik in der Beschreibung der Beispiele

Wenn nicht anders angegeben, sind die Daten als Mittelwert ± SD ausgedrückt. Die statistische Analyse wurde mit GraphPad Prism (GraphPad Software, San Diego, CA, USA; Version 8.0.2) durchgeführt. Zum Vergleich zweier Versuchsgruppen wurden zweiseitige t-Tests durchgeführt. Für den Vergleich von mehr als zwei Versuchsgruppen wurde eine ANOVA mit Tukey's multiplen Testkorrekturen durchgeführt. Signifikante p-Werte sind mit * (≤0,05), ** (≤0,01) oder *** (≤0,001) gekennzeichnet.

### h) Vergleichsbeispiele für Erzeugnisse, die ein Nahrungsergänzungsmittel sind

Ein Beispiel eines Erzeugnisses zur oralen Einnahme wird in der folgenden Tabelle dargestellt:

| | |
|---|---|
| Kohlenhydrate | 45 g |
| Ballaststoffe | 25 g |
| Fett | < 1 g |
| Protein | < 1 g |
| Salz | < 0,5 g |
| Zink | 0,15 g |
| Biotin | 0,6 mg |
| Folsäure | 4,0 mg |
| Vitamin B6 | 20 mg |
| Extrakt aus Blättern der Preiselbeere | 4,5 g |
| Natürliche Aromen | 3,5 g |
| Docosahexaensäure | 1,5 g |
| Eicosapentaensäure | 70 mg |
| Coenzym Q10 | 120 mg |
| Weitere Hilfsstoffe | Ad 100 g |

Es handelt sich um ein Nahrungsergänzungsmittel. Die Tagesportion beträgt 10 g.

In einem weiteren Beispiel werden statt 4,5 g des Extraktes aus Blättern der Preiselbeere 2,25 g des Extraktes aus Blättern der Preiselbeere und 2,25 g eines Extraktes aus Blättern der Brombeere zugegeben.

In einem weiteren Beispiel werden statt 4,5 g des Extraktes aus Blättern der Preiselbeere 4,5 g eines Extraktes aus Blättern der Brombeere zugegeben.

In einem weiteren Beispiel werden statt 4,5 g des Extraktes aus Blättern der Preiselbeere 4,5 g eines Odermennigblätterextraktes zugegeben.

## Patentansprüche

1. Erzeugnis zur oralen Einnahme, wobei das Erzeugnis einen Extrakt aus den Blättern der Preiselbeerpflanze *(Vaccinium vitis-idea)* und/oder einen Extrakt aus den Blättern der Brombeerpflanze *(Rubus fructicosus)* enthält, wobei der Extrakt oder die Extrakte durch ein Extraktionsverfahren mit einem wässrigen Extraktionsmittel hergestellt wurden, und wobei das Erzeugnis ein Nahrungsergänzungsmittel ist, **dadurch gekennzeichnet, dass** das Erzeugnis pro 100 g des Erzeugnisses 4 mg bis 200 mg eines Extraktes aus dem Pfeffer *(Piper nigrum)* enthält.

2. Erzeugnis zumindest nach Anspruch 1, wobei das Erzeugnis den Extrakt aus den Blättern der Preiselbeerpflanze *(Vaccinium vitis-idea)* enthält.

3. Erzeugnis nach einem der vorherigen Ansprüche 1 oder 2, wobei das Erzeugnis den Extrakt aus den Blättern der Brombeerpflanze *(Rubus fructicosus)* enthält.

4. Erzeugnis zumindest nach einem der vorherigen Ansprüche 1 bis 3, wobei das Erzeugnis neben dem Extrakt aus der Preiselbeerpflanze *(Vaccinium vitis-idea)* und/oder dem Extrakt aus der Brombeerpflanze *(Rubus fructicosus)* und dem Extrakt aus dem Pfeffer einen oder mehrere Inhaltsstoffe ausgewählt aus Zink, Biotin, Omega-3-Fettsäuren, Coenzym Q10, Berberin, Lutein, Zeaxanthin, einem Extrakt aus Broccoli, einem Extrakt aus der Zwiebel, einem Extrakt aus dem Apfel, einem Extrakt aus der Acerola, einem Extrakt aus der Tomate, einem Extrakt aus der Kurkuma, einem Extrakt aus dem Knoblauch, einem Extrakt aus dem Basilikum, einem Extrakt aus dem Oregano, einem Extrakt aus dem Zimtbaum, einem Extrakt aus der Karotte, einem Extrakt aus dem Holunder, einem Extrakt aus der Johannisbeere, einem Extrakt aus der Heidelbeere, einem Extrakt aus der Himbeere, einem Extrakt aus der Apfelbeere, einem Extrakt aus dem Spinat, einem Extrakt aus der Kirsche, einem Extrakt aus der Heidelbeere und einem Extrakt aus dem Rosenkohl enthält.

5. Erzeugnis zumindest nach einem der vorherigen Ansprüche 1 bis 4, wobei das Erzeugnis pro 100 g des Erzeugnisses ein oder mehrere Bestandteile enthält, die ausgewählt sind aus 0,25 mg bis 2 g einer Curcuminverbindung und 4 mg bis 200 mg eines Extraktes aus dem Ingwer.

6. Erzeugnis zumindest nach Anspruch 5, wobei das Erzeugnis pro 100 g des Erzeugnisses 0,25 mg bis 2 g einer Curcuminverbindung und 4 mg bis 200 mg eines Extraktes aus dem Ingwer enthält.

7. Erzeugnis zumindest nach einem der vorherigen Ansprüche, wobei das Erzeugnis pro 100 g des Erzeugnisses 0,4 g bis 6 g eines Extraktes aus rotem Ginseng enthält.

## Claims

1. Product for oral intake, wherein the product contains an extract of the leaves of the lingonberry plant (*Vaccinium vitis-idea*) and/or an extract of the leaves of the blackberry plant (*Rubus fructicosus*), wherein the extract or the extracts are produced by an extraction method using an aqueous extracting agent, and wherein the product is a nutritional supplement, **characterized in that** the product contains 4 mg to 200 mg of an extract of pepper (*Piper nigrum*) per 100 g of the product.

2. Product at least as claimed in claim 1, wherein the product contains the extract of the leaves of the lingonberry plant (*Vaccinium vitis-idea*).

3. Product as claimed in either one of preceding claims 1 or 2, wherein the product contains the extract of the leaves of the blackberry plant (*Rubus fructicosus*).

4. Product at least as claimed in one of preceding claims 1 to 3, wherein the product contains, in addition to the extract of the lingonberry plant (*Vaccinium vitis-idea*) and/or an extract of the blackberry plant (*Rubus fructicosus*) and the extract of pepper, one or more ingredients selected from zinc, biotin, omega-3 fatty acids, coenzyme Q10, berberine, lutein, zeaxanthin, an extract of broccoli, an extract of onion, an extract of apple, an extract of acerola, an extract of tomato, an extract of turmeric, an extract of garlic, an extract of basil, an extract of oregano, an extract of cinnamon tree, an extract of carrot, an extract of elder, an extract of currant, an extract of blueberry, extract of raspberry, an extract of chokeberry, an extract of spinach, an extract of cherry, an extract of blueberry, and an extract of Brussels sprouts.

5. Product at least as claimed in one of preceding claims 1 to 4, wherein the product contains, per 100 g of the product, one or more components selected from 0.25 mg to 2 g of a curcumin compound and 4 mg to 200 mg of an extract of ginger.

6. Product at least as claimed in claim 5, wherein the product contains, per 100 g of the product, 0.25 mg to 2 g of a curcumin compound and 4 mg to 200 mg of an extract of ginger.

7. Product at least as claimed in one of the preceding claims, wherein the product contains, per 100 g of the product, 0.4 g to 6 g of an extract of red ginseng.

## Revendications

1. Produit à prendre par voie orale, lequel produit contient un extrait de feuilles d'airelle rouge (*Vaccinium vitis-idea*) et/ou un extrait de feuilles de ronce commune (*Rubus fructicosus*), l'extrait ou les extraits étant fabriqués par un procédé d'extraction avec un agent d'extraction aqueux et le produit étant un complément alimentaire, **caractérisé en ce que** le produit contient, pour 100 g de produit, de 4 mg à 200 mg d'un extrait de poivre (*Piper nigrum*).

2. Produit selon au moins la revendication 1, lequel produit contient l'extrait de feuilles d'airelle rouge (*Vaccinium vitis-idea*).

3. Produit selon l'une des revendications 1 ou 2, lequel produit contient l'extrait de feuilles de ronce commune (*Rubus fructicosus*).

4. Produit selon au moins une des revendications 1 à 3, lequel produit contient, outre l'extrait de feuilles d'airelle rouge (*Vaccinium vitis-idea*) et/ou l'extrait de feuilles de ronce commune (*Rubus fructicosus*) et l'extrait de poivre, un ou plusieurs ingrédients choisis parmi le zinc, la biotine, les acides gras oméga-3, la coenzyme Q10, la berbérine, la lutéine, la zéaxanthine, un extrait de brocoli, un extrait d'oignon, un extrait de pomme, un extrait d'acérola, un extrait de tomate, un extrait de curcuma, un extrait d'ail, un extrait de basilic, un extrait d'origan, un extrait de cannellier, un extrait de carotte, un extrait de sureau, un extrait de groseille, un extrait de myrtille, un extrait de framboise, un extrait d'aronia, un extrait d'épinard, un extrait de cerise, un extrait de myrtille et un extrait de chou-fleur.

5. Produit selon au moins une des revendications 1 à 4, lequel produit contient, pour 100 g du produit, un ou plusieurs ingrédients choisis parmi 0,25 mg à 2 g d'un composé de curcumine et 4 mg à 200 mg d'un extrait de gingembre.

6. Produit selon au moins la revendication 5, lequel produit contient, pour 100 g de produit, de 0,25 mg à 2 g d'un composé de curcumine et entre 4 mg et 200 mg d'un extrait de gingembre.

7. Produit selon au moins une des revendications précédentes, lequel produit contient, pour 100 g de produit, de 0,4 g à 6 g d'un extrait de ginseng rouge.
